# EUROPEAN PATENT SPECIFICATION

(11) **EP 1 520 529 B1**
(45) Date of publication and mention of the grant of the patent: **02.11.2006**
(21) Application number: 04256038.3
(22) Date of filing: 30.09.2004
(51) Int. Cl.: A61B 17/11

(54) **Unfolding anastomosis ring device**
Entfaltbarer Anastomosering
Anneau dépliable d'anastomose

(30) Priority: 30.09.2003 US 675091
(43) Date of publication of application: 06.04.2005
(73) Proprietor: ETHICON ENDO-SURGERY, INC., Cincinnati, Ohio 45242 (US)
(72) Inventor: Beaupre, Jean, Cincinnati Ohio 45242 (US)
(74) Representative: Tunstall, Christopher Stephen

(56) References cited:
- WO-A-00/12832
- WO-A-99/21491
- WO-A-03/000142
- US-A1- 2003 109 893

## Description

### Field of the Invention

The present invention relates, in general, to devices for surgically modifying organs and vessels. More particularly, it relates to anastomosis devices for joining two organs such as, for example, two separate lengths of small bowel to each other, a section of small bowel to the stomach, or the common bile duct to the duodenum in a procedure called a choledochoduodenostomy. Vascular anastomosis could be performed as well.

### Background of the Invention

Creating an anastomosis, or the surgical formation of a passage between two normally distinct vessels, is a critical step of many surgical procedures. This is particularly true of gastric bypass procedures in which two portions of small intestine are joined together and another portion of small intestine is joined to the stomach of the patient. This is also true of surgery to alleviate blockage in the common bile duct by draining bile from the duct to the small intestine during surgery for pancreatic cancer.

The percentage of the world population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacologic methods have all been tried, and though temporarily effective, failed to correct the condition. Further, introducing an object in the stomach, such as an esophago-gastric balloon, to fill the stomach have also been used to treat the condition; however, such approaches tend to cause irritation to the stomach and are not effective long-term.

Surgical treatments of morbid obesity have been increasingly used with greater success. These approaches may be generalized as those that reduce the effective size of the stomach, limiting the amount of food intake, and those that create malabsorption of the food that it is eaten. For instance, some patients benefit from adjustable gastric bands (AGB) that are advantageously laparoscopically placed about the stomach to form a stoma of a desired size that allows food to fill an upper portion of the stomach, causing a feeling of satiety. To allow adjustment of the size of the stoma after implantation, a fluid conduit communicates between an inwardly presented fluid bladder of the AGB to a fluid injection port subcutaneously placed in front of the patient's sternum. A syringe needle may then inject or withdraw fluid as desired to adjust the AGB.

Although an effective approach to obesity for some, other patients may find the lifestyle changes undesirable, necessitated by the restricted amount of food intake. In addition, the medical condition of the patient may suggest the need for a more permanent solution. To that end, surgical approaches have been used to alter the portions of the stomach and/or small intestine available for digesting food. Current methods of performing a laparoscopic anastomoses for a gastric bypass include stapling, suturing, and placing biofragmentable rings, each having significant challenges. For instance, suturing is time consuming, as well as being technique and dexterity dependent. Stapling requires placement of an anvil, which is a large device that cannot be introduced through a trocar port. Having to introduce the port through a laparotomy presents an increased incidence of wound site infection associated with intralumenal content being dragged to the laparotomy entry site.

As an example of the latter approach, in U.S. Pat. No. 6,543,456 a method for gastric bypass surgery includes the insertion of proximal and distal anastomosis members (e.g., anvils) transorally with grasping forceps. The stomach and the small intestine are transected endoscopically by a surgical severing and stapling instrument to create a gastric pouch, a drainage loop, and a Roux limb. An endoscopically inserted circular stapler attaches to the distal anastomosis member to join the drainage loop to a distal portion of the intestine, and the circular stapler attaches to the proximal anastomosis member to join the Roux limb to the gastric pouch. Thereafter, the anastomosis members are removed to create an orifice between joined portions of the stomach and intestine. This method reduces the number of laparoscopic ports, avoids a laparoscopic insertion of an anastomosis instrument (e.g., circular stapler) into an enlarged surgical port, and eliminates the need for an enterotomy and an enterotomy closure.

For many anastomoses, surgeons use circular staplers, linear staplers, or manual sutures. However, to reduce incision size and to make the surgical process less technically demanding and time consuming, an anastomotic device that deforms to hold tissue portions together when the device is ejected from a constraining enclosure has been described. Such an approach is described in PCT application WO 03/000142 upon which the two part form of claim 1 is based describes such a device. Therein, gastrointestinal or enteric (including biliary) anastomosis is achieved by insertion of a sheath that perforates the walls of two tissue passages, such as the stomach and small intestine. A three-dimensional woven tube of wire of having a thermal shape memory effect (SME) ("generally-known nitinol ring device") is presented by a cannula of the sheath on both sides of the openings. Deployment of the woven tube causes the outer loops or ends of the tube to fold or loop back to hold the luminal interface of the anastomosis site in apposition. Thereby, the need for a mechanical compression component in a delivery system is reduced or avoided, reducing the size and complexity of the delivery device.

The anastomotic device disclosed in WO 03/000142 is constrained by a retractable sheath to an advantageous small-diameter tubular shape. A surgeon applies the anastomotic device by maneuvering the sheath through the tissue portions requiring anastomosis and retracting the sheath. Retracting the sheath removes the constraint on the device, allowing the device to assume a roughly hourglass shape. The larger ends of the hourglass shape hold the two tissue portions together in an effective anastomosis.

The constrained anastomotic device, which may be made of a shape memory material such as nitinol, exerts a force against the inner diameter of the sheath and tends to warp towards its roughly hourglass-shaped deployed position. When the sheath is retracted proximally, the forces generated by the device in transition from a tubular shape to an hourglass shape urge the anastomotic device distally. This device movement makes surgical control harder to achieve when placing the device through the otomies of two tissue portions requiring anastomosis.

While the generally-known nitinol ring device is a significant advancement in the treatment of morbid obesity, it is believed that further improvements would be desirable. For instance, the continuous interlocking petals are difficult to manufacturer, especially since the depicted woven tube is of a continuous wire loop bent into a pattern of interlocking triangles that are hand woven from two wire strands and the four free ends connected to one another.

Consequently, there is a general need for an approach to making an anastomosis ring device that can be used in existing trocar ports (e.g., 12 mm size) and that reliably and effectively creates an anastomotic attachment between lumens, eliminate the need for surgical stapling and suturing to form an anastomosis.

### Brief Summary of the Invention

According to the invention an anastomotic ring device is provided as in appended claim 1. Advantageous embodiments are defined in the dependent claims.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIGURE 1 is perspective view of an anastomotic ring device assembled from a plurality of arcuate petals.

FIGURE 2 is a perspective view of one longitudinal half, or "crown" of the anastomotic ring device of FIG. 1 being assembled onto a fixture.

FIGURE 3 is a perspective view of the two halves, or a crown and mirrored or inverted crown prior to attachment one to the other.

FIGURE 4 is a perspective view of an applier capable of implantation, actuation and deployment of an anastomotic ring device of FIG. 1, which is retained in an unactuated, cylindrical shape.

FIGURE 5 is a detail view of the distal tip including an actuating member and piercing tip of the applier of FIG. 4 retaining the anastomotic ring device of FIG. 1 by gripping each point of each respective petal.

FIGURE 6 is a perspective the applier of FIG. 4 actuating with opposing, compressive longitudinal actuation motions the anastomotic ring device of FIG. 1 into an actuated hollow rivet, or hour glass, shape to form an anastomotic attachment.

FIGURE 7 is a detail view of the distal tip of the applier of FIG. 6, depicting the actuated anastomotic ring device having been released from the actuating member in preparation for deployment from the applier.

FIGURE 8 is a perspective view of hinged anastomotic ring device assembled in an actuated condition from a plurality of molded arcuate petals.

FIGURE 9 is a perspective view of one molded arcuate petal.

FIGURE 10 is a perspective view of one half of the hinged anastomotic ring device of FIG. 8 assembled into hinged petals.

FIGURE 11 is a perspective view of the applier of FIG. 4 actuating to implant the hinged anastomotic ring device of FIG. 8.

FIGURE 12 is a detail view of the distal tip of the applier of FIG. 11 depicting the actuated hinged anastomotic ring device having been released from the actuating member in preparation for deployment from the applier.

### Detailed Description of the Invention

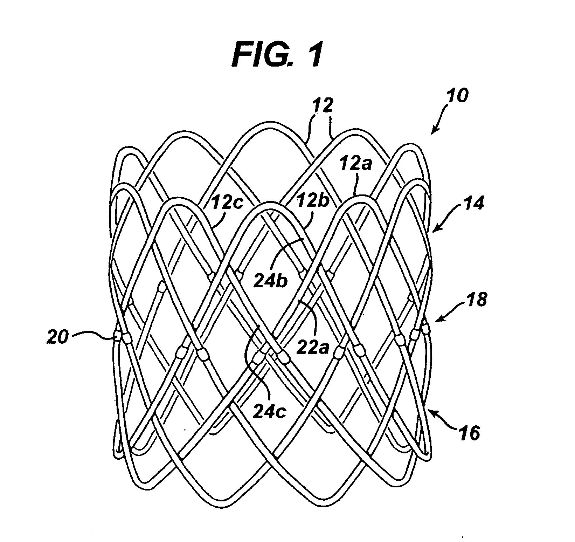

Turning to the Drawings, wherein like numerals denote like components throughout the several views, FIG. 1 depicts an anastomotic ring device 10 in its generally cylindrical, unactuated condition, with its woven tube of strands resembling the interweaving of a chain link fence. In the illustrative embodiment, a plurality of arcuate members, or petals, 12 are assembled in a longitudinal half, or crown 14, intended to be on one side of an anastomotic attachment site, with a similar but inverted or mirrored crown 16, intended to be on the other side of the anastomotic attachment site.

Both halves or crowns 14, 16 are attached at a circular midpoint 18 such that the plurality of arcuate members 12 resemble a plurality woven sinusoids. At the midpoint 18, attachments 20 respectively between pair of end 22, 24 from an arcuate member 12 in the top crown 14 is made to a respective end 22, 24 of an arcuate member 12 in the bottom crown 16. A non-exclusive list of couplings 20 include snap fits, glue, ultrasonic welding, thermal adhesives, etc.

In the illustrative ring device of FIG. 1, ten arcuate members 12 are included in each crown 14, 16. Each arcuate member is woven with two arcuate members to each side and attached to two arcuate members in the other half. For instance, for arcuate member 12a, a left end 22a passes in front (outside) of a right end 24b of a left adjacent arcuate member 12b and then passing behind a right end 24c of a farther left arcuate member 12c, with the pattern repeated about the circumference of the crown 14. It should be appreciated that this number of arcuate members and this degree of interweaving is illustrative and that other patterns consistent with aspects of the invention may be used.

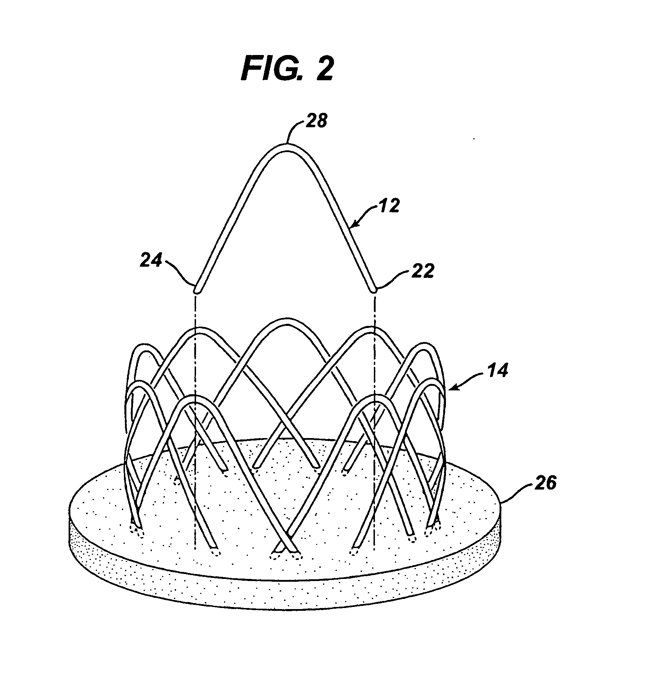

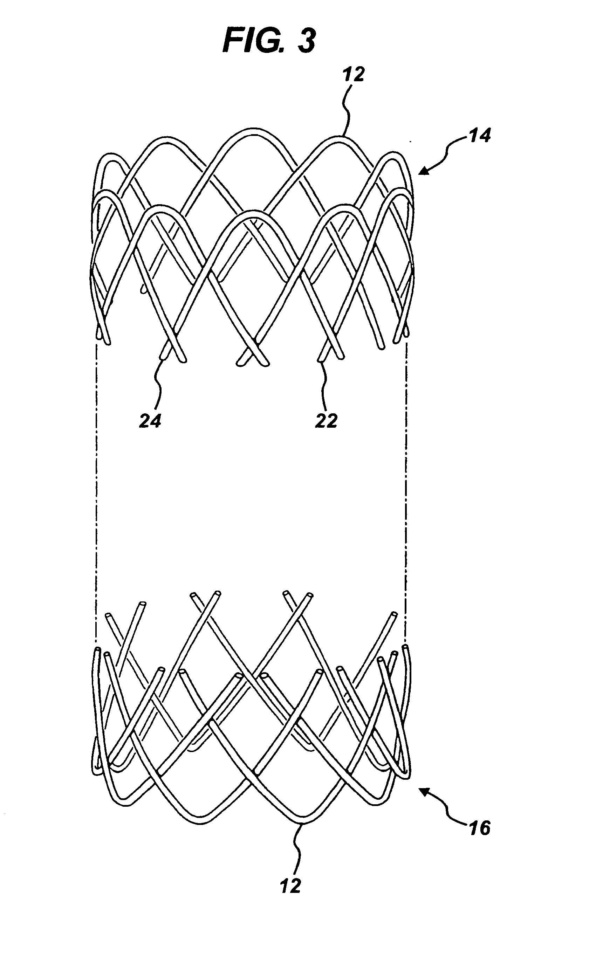

In FIG. 2, assembly of the crown 14 is depicted, illustrating how the plurality of arcuate members 12 facilitates economical manufacture that may be performed by automated mechanisms. In this depiction, a fixture, or disk, 26 holds the plurality of arcuate members 12 until the crown 14 is complete, specifically locating each pair of ends 22, 24 of each arcuate member 12 for attachment to the other crown 16 (shown in FIG. 3). Moreover, the fixture 26, ensures that each curved point 28, from which each end 22, 24 diverges, is equidistantly spaced about the crown 14 and evenly extending for engagement by an applier.

### Anastomotic Ring Device Applier.

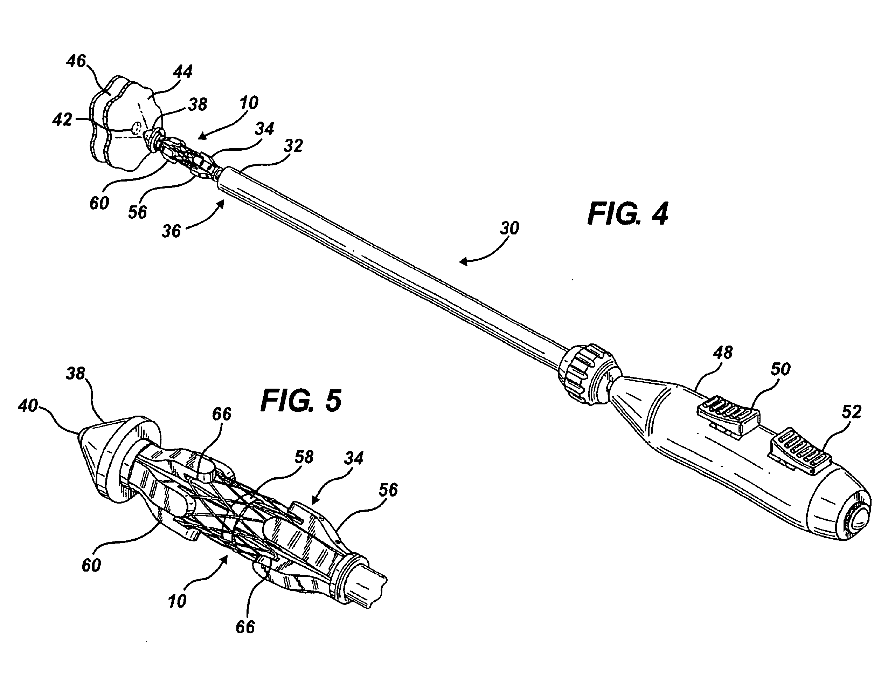

In FIGS. 4-7, an illustrative applier 30 has the anastomotic ring device 10 advantageously retained in a generally cylindrical shape (FIGS. 4-5) distal to an outer tube 32 upon a molded actuation member 34 forming a cannula 36 that distally terminates in a flared tip 38. This flared tip 38 presents a distal piercing surface 40 to form an anastomotic opening 42 through apposite tissue walls 44, 46 of two gastrointestinal passages.

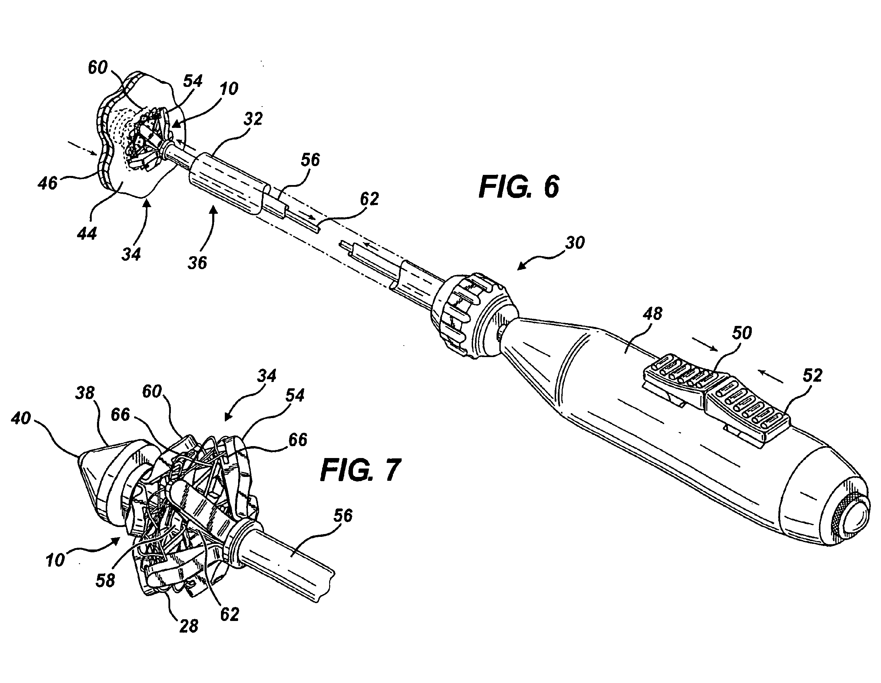

With particular reference to FIG. 6, a handle 48, proximal to the cannula 36, includes a pair of longitudinally aligned triggers 50, 52. The proximal trigger 50, shown at its most proximal, unfired position, is coupled to proximal leaves 54 of the molded actuation member 34 via an intermediate tube 56 of the cannula 36. Distal movement of the proximal trigger 50 thus causes longitudinal distal movement of the intermediate tube 56 and proximal leaves 54, which outwardly actuate like an umbrella by a hinged relationship to a central portion 58 of the molded actuation member 34. (Unlike an umbrella, the "top" is brought toward the center rather than the converse.)

Similarly, the distal trigger 52, shown at its most distal, unfired position, is coupled to distal leaves 60 of the molded actuation member 34 via an internal rod 62 that is coupled for movement within the intermediate tube 56. Proximal movement of the distal trigger 52 causes longitudinal proximal movement of the rod 62 and distal leaves 60 of the molded actuation member 34, which outwardly actuate by a hinged relationship to the central portion 58.

In FIGS. 6-7, the triggers 50, 52 have been slid toward one another to actuate the molded actuating member 34. Specifically, the distal trigger 52 has been moved proximally, causing similar distal movement of the internal rod 62, the distal terminating end of the latter being attached to flared tip 38. The flared tip 38 thus moves toward the distal end of the intermediate tube 56. The proximal trigger 50 has been moved distally, moving intermediate tube 56 also distally. The molded actuating member 34 is compressed between the inwardly moving flared tip 38 and intermediate tube 56. The distal leaves 64 actuate lateral to the longitudinal axis, and move toward and interdigitate with the proximal leaves 54. This movement expedites actuating of an anastomotic ring device 10.

In use, the flared tip 38 of the applier 30 is inserted through a trocar port into a tissue passage that has been placed proximate to another tissue passage that are to be anastomotically joined (See FIGS. 4-5). The flared tip 38 and a distal half of the molded actuating member 34 and anastomotic ring device 10 are inserted through an anastomotic opening 42 formed therebetween and then the applier 30 is actuated. With particular reference to FIGS. 6-7, the proximal and distal leaves 54, 60 are shown as having gripping slots 66 that grip respective curved points 28 of each arcuate member or petals 12 of the anastomotic ring device 10, especially in its unactuated, generally cylindrical shape. These gripping slots 66 assist in preventing the anastomotic ring device 10 from slipping off of the applier 30 or being inappropriately placed thereon for actuation until fully actuated, forming the anastomotic ring device 10 into a hollow rivet shape or hourglass shape to form the anastomotic attachment between tissue walls 44, 46. The fully actuated proximal and distal leaves 54, 60 cause the curved points 28 to disengage from the gripping slots 66. Thereafter, the applier 30 is returned to an unactuated condition and the actuated anastomotic ring device 12 deployed by withdrawing the flared tip 38 from the anastomotic opening 42 and ring device 12.

It should be appreciated that the unactuated anastomotic ring device 10 may be formed from nitinol and temperature treated to create a Shape Memory Effect that would cause self-actuation after implantation to a hollow rivet or hourglass shape, thus allowing generally known appliers to be used. However, as described above and in more detail in EP 1520530 US2005070921 such actuation is enhanced or performed entirely by the applier 30 capable of causing the rapid actuation of the anastomotic ring device 10, thus allowing other materials to be used as well as nitinol. Moreover, the ability to cause actuation with an applier 30 enables the use of ring devices with no inherent actuating ability.

### Hinged anastomotic ring device.

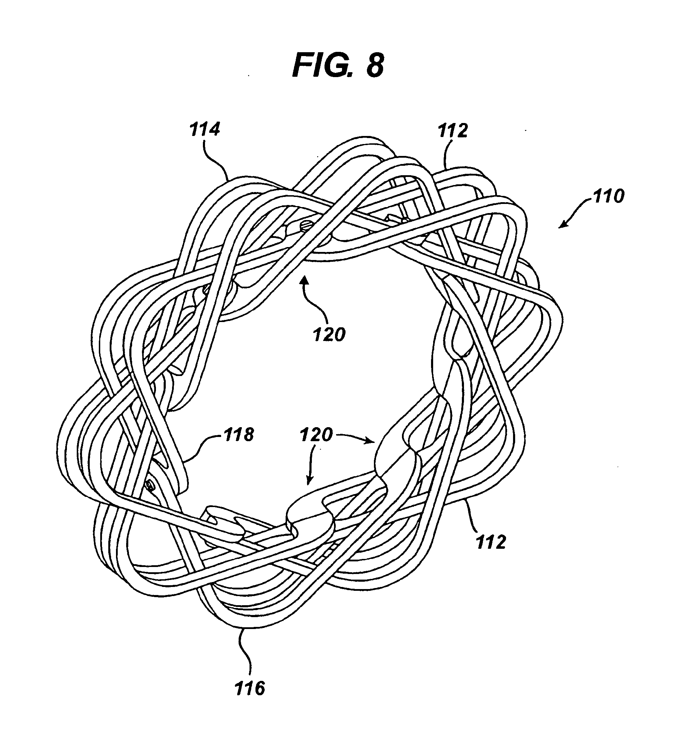

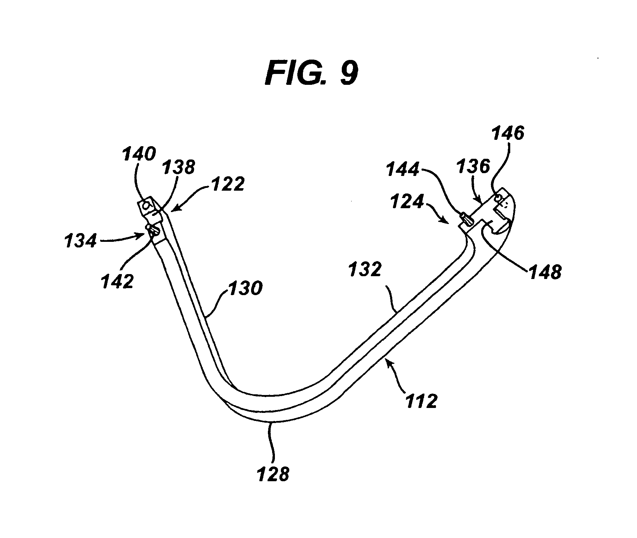

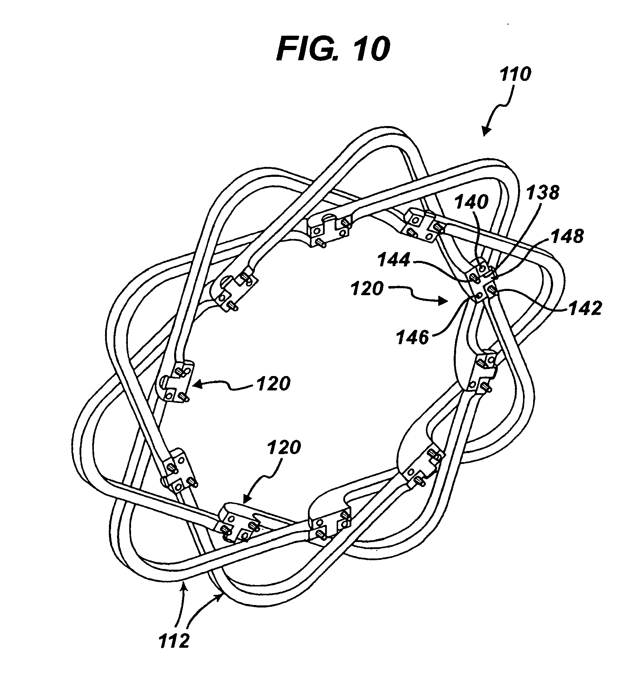

For instance, in FIGS. 8-10, another anastomotic ring device 110 is formed from molded arcuate members 112 that show further advantages of forming two crowns 114, 116 with attachments 120 at a circular midpoint 118. With particular reference to FIG. 9, each arcuate member 112 has a first end 122 and a second end 124 convergingly joined at a curved point 128. Each first end 122, 124 bends perpendicularly to their respective elongate shafts 130, 132 presenting respectively pin hinge receiving surface 134 and a pin hinge surface 136. The pin hinge receiving surface 134 includes a lateral half cylinder recess 138 interposed between a distally presented female attachment feature 140 and a proximally disposed male attachment feature 142. The pin hinge surface 136 includes a half pin 144 interposed between a distally disposed female attachment feature 146 and a proximally disposed male attachment feature 148.

These first and second ends 122, 124 of the arcuate member 112 in one crown 114 facilitate a rigid attachment at attachment 120 to rotated identical arcuate members of the other crown 116. The joined first ends 122 between the two arcuate members 112 forms a through hole of the two half cylinder recesses 138 that receive a pin hinge formed from two half pins 144 formed from two second ends 124. Thus each arcuate member 112 is interwoven with its two adjacent arcuate members 112, moves in concert with its two attached arcuate members 112 in the other crown 116 and is hingedly connected to arcuate members 112 that are on the other side to the adjacent arcuate members 112.

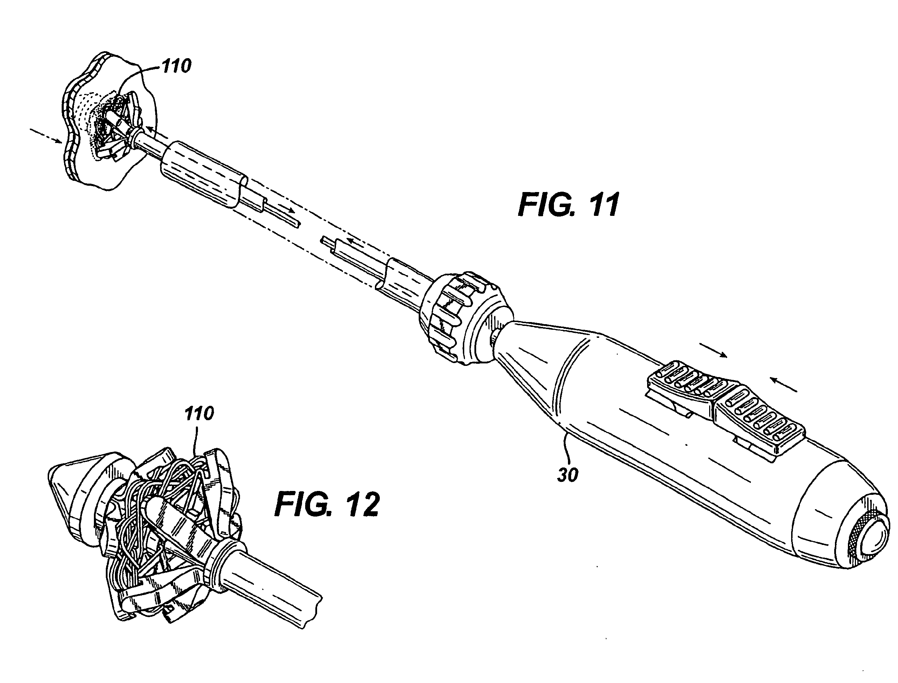

Sufficient friction exists in the hinged connection between arcuate members at the midpoint 118 that when placed in position, such as depicted in FIGS. 11-12 by an applier 30, the anastomotic ring device 110 tends to stay in its actuated position. Alternatively, the anastomotic ring device 110 is intended to maintain the anastomotic opening and requires a secondary fastening element to remain in the actuated position, such as sutures fastening arcuate members 112 to the tissue and to one another.

Although such molded arcuate members 112 may be assembled in an unactuated, cylindrical fashion as previously described above for the wire anastomotic ring device 10, in FIG. 9, it is shown how one crown 114 may be formed in an actuated configuration, readily prepared to accept individual arcuate members 112 of the other crown or a fully assembled bottom crown 116.

Each molded arcuate member may be formed from a bioabsorbable material, such as a biofragmentable polymer mixture that eventually passes out of the digestive tract.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, a molded arcuate member consistent with aspects of the invention may form a hinged relationship rather than a rigid attachment to respective arcuate members of the inverted crown.

As a further example, an anastomosis ring device may include a circular fixture or band at its midpoint for attaching the arcuate members that remains part of the anastomosis ring device, intended to sit at a tissue juncture of the anastomosis.

## Claims

1. An anastomosis ring (10) transformable from a first insertion state in the form of a woven cylindrical tube to a second deployed state in the form of a woven hollow rivet, comprising:
a first plurality of arcuate members (12), which in the first state are arranged in a cylindrical crown shape (14) with each arcuate member having legs overlapping at least one adjacent arcuate member; and
a second plurality of arcuate members, which in the first state are arranged in an inverted cylindrical crown shape (16) with each arcuate member having legs overlapping at least one adjacent arcuate member of the second plurality and connected to a leg of an arcuate member of the first plurality;
wherein in the second state each arcuate member is outwardly deflected from a longitudinal axis of its respective cylindrical crown toward apposing arcuate members of the other cylindrical crown;
**characterised in that,**
the arcuate members of the first plurality are discrete; and
the arcuate members of the second plurality are discrete.

2. The anastomosis device of claim 1, wherein the arcuate members comprise a shape memory effect alloy.

3. The anastomosis device of claim 1, wherein the legs of the arcuate members of the first plurality are attached to the respective arcuate member of the second plurality by a connecting member.

4. The anastomosis device of claim 3, wherein the connecting members comprises a selected one of a group consisting of a snap fit, a glue, an ultrasonically welded portion, and a thermally melted polymer.

5. The anastomosis device of claim 3, wherein the legs of the arcuate members of the first plurality are attached to the respective arcuate member of the second plurality by a rigid connecting member, a petal formed by the first arcuate member actuating generally in a plane with the respective attached arcuate members pivoting about a cylindrical midpoint of the anastomosis device.

6. The anastomosis device of claim 5, wherein each leg is further hingedly coupled at the circular midpoint of the anastomosis device to at least one other leg.

7. The anastomosis device of claim 3, wherein the legs of the arcuate members of the first plurality are attached to the respective arcuate member of the second plurality by a pivotal connecting member.

8. The anastomosis device of claim 3, wherein the connecting member comprises a band at a midpoint of the device connected to each arcuate member.

9. The anastomosis ring device of claim 1, wherein the arcuate members arranged into two crowns are attached to one another to present petals circumferentially hinged at a cylindrical midpoint of the anastomosis device, each arcuate member comprising a pair of diverging connected legs.

## Patentansprüche

1. Anastomosering (10), der aus einem ersten Einsetzzustand in der Form eines gewebten zylindrischen Rohres in einen zweiten Einsatzzustand in der Form einer gewebten Lochniete umwandelbar ist, umfassend:
eine erste Anzahl von bogenförmigen Elementen (12), die im ersten Zustand in einer Gestalt (14) einer zylindrischen Krone angeordnet sind, wobei jedes bogenförmige Element Schenkel aufweist, die wenigstens ein benachbartes bogenförmiges Element überlappen, und
eine zweite Anzahl von bogenförmigen Elementen, die im ersten Zustand in der Gestalt (16) einer umgedrehten zylindrischen Krone angeordnet sind, wobei jedes bogenförmige Element Schenkel aufweist, die wenigstens ein benachbartes bogenförmiges Element der zweiten Anzahl überlappen und mit einem Schenkel eines bogenförmigen Elements der ersten Anzahl verbunden sind,
worin im zweiten Zustand jedes bogenförmige Element von einer Längsachse seiner jeweiligen zylindrischen Krone in Richtung auf angelagerte bogenförmige Elemente der anderen zylindrischen Krone umgebogen ist,
**dadurch gekennzeichnet, daß** die bogenförmigen Elemente der ersten Anzahl diskret sind und die bogenförmigen Elemente der zweiten Anzahl diskret sind.

2. Anastomosering nach Anspruch 1, **dadurch gekennzeichnet, daß** die bogenförmigen Elemente eine Formgedächtniseffektlegierung umfassen.

3. Anastomosering nach Anspruch 1, **dadurch gekennzeichnet, daß** die Schenkel der bogenförmigen Elemente der ersten Anzahl an dem jeweiligen bogenförmigen Element der zweiten Anzahl durch ein Verbindungselement angebracht sind.

4. Anastomosering nach Anspruch 3, **dadurch gekennzeichnet, daß** die Verbindungselemente einen/eine/eines umfassen, der/die/das aus einer Gruppe ausgewählt ist, die aus einem Schnappverschluß, einem Klebstoff, einem mittels Ultraschall geschweißten Abschnitt und einem thermisch geschmolzenem Polymer besteht.

5. Anastomosering nach Anspruch 3, **dadurch gekennzeichnet, daß** die Schenkel der bogenförmigen Elemente der ersten Anzahl an dem jeweiligen bogenförmigen Element der zweiten Anzahl durch ein starres Verbindungselement angebracht sind, wobei ein von dem ersten bogenförmigen Element gebildetes Blatt allgemein in einer Ebene wirkt, wobei die jeweiligen angebrachten bogenförmigen Elemente um einen Zylindermittelpunkt des Anastomoserings schwenken.

6. Anastomosering nach Anspruch 5, **dadurch gekennzeichnet, daß** jeder Schenkel ferner an dem Kreismittelpunkt des Anastomoserings mit wenigstens einem weiteren Schenkel schwenkbar gekoppelt ist.

7. Anastomosering nach Anspruch 3, **dadurch gekennzeichnet, daß** die Schenkel der bogenförmigen Elemente der ersten Anzahl an dem jeweiligen bogenförmigen Element der zweiten Anzahl durch ein schwenkbares Verbindungselement angebracht sind.

8. Anastomosering nach Anspruch 3, **dadurch gekennzeichnet, daß** das Verbindungselement ein Band an einem Mittelpunkt des Rings umfaßt, das mit jedem bogenförmigen Element verbunden ist.

9. Anastomosering nach Anspruch 1, **dadurch gekennzeichnet, daß** die in zwei Kronen angeordneten bogenförmigen Elemente aneinander angebracht sind, um Blätter zu bilden, die an einem Zylindermittelpunkt des Anastomoserings umlaufend schwenkbar sind, wobei jedes bogenförmige Element ein Paar divergierende Anschlußschenkel aufweist.

## Revendications

1. Anneau d'anastomose (10) transformable entre un premier état d'insertion sous la forme d'un tube cylindrique treillissé et un second état déployé sous la forme d'un rivet creux treillissé, comprenant :
une première pluralité d'éléments arqués (12) qui, dans le premier état, sont agencés en forme de couronne cylindrique (14), chaque élément arqué ayant des pattes chevauchant au moins un élément arqué adjacent ; et
une seconde pluralité d'éléments arqués qui, dans le premier état, sont agencés en forme de couronne cylindrique inversée (16), chaque élément arqué ayant des pattes chevauchant au moins un élément arqué adjacent de la seconde pluralité et raccordées à un élément arqué de la première pluralité ;
dans le second état, chaque élément arqué étant dévié, vers l'extérieur, d'un axe longitudinal de sa couronne cylindrique respective vers les éléments arqués en apposition de l'autre couronne cylindrique ;
**caractérisé en ce que**
les éléments arqués de la première pluralité sont discrets ; et
les éléments arqués de la seconde pluralité sont discrets.

2. Dispositif d'anastomose selon la revendication 1, dans lequel les éléments arqués comprennent un alliage à mémoire de forme.

3. Dispositif d'anastomose selon la revendication 1, dans lequel les pattes des éléments arqués de la première pluralité sont rattachées à l'élément arqué respectif de la seconde pluralité par un élément de raccord.

4. Dispositif d'anastomose selon la revendication 3, dans lequel l'élément de raccord comprend un élément choisi dans le groupe constitué par un emboîtement, une colle, une partie soudée par ultrasons et un polymère à fusion thermique.

5. Dispositif d'anastomose selon la revendication 3, dans lequel les pattes des éléments arqués de la première pluralité sont rattachées à l'élément arqué respectif de la seconde pluralité par un élément de raccord rigide, un pétale formé par le premier élément arqué agissant globalement dans un plan avec les éléments arqués rattachés respectifs pivotant autour d'un point médian cylindrique du dispositif d'anastomose.

6. Dispositif d'anastomose selon la revendication 5, dans lequel chaque patte est en outre couplée de manière articulée, au niveau du point médian circulaire du dispositif d'anastomose, à au moins une autre patte.

7. Dispositif d'anastomose selon la revendication 3, dans lequel les pattes des éléments arqués de la première pluralité sont rattachées à l'élément arqué respectif de la seconde pluralité par un élément de raccord pivotant.

8. Dispositif d'anastomose selon la revendication 3, dans lequel l'élément de raccord comprend une bague au niveau d'un point médian du dispositif en connexion avec chaque élément arqué.

9. Dispositif d'anneau d'anastomose selon la revendication 1, dans lequel les éléments arqués agencés en deux couronnes sont rattachés les uns aux autres pour présenter des pétales articulés circonférentiellement en un point médian cylindrique du dispositif d'anastomose, chaque élément arqué se composant d'une paire de pattes divergentes raccordées.
